# EUROPEAN PATENT APPLICATION

(11) **EP 3 127 479 A1**
(43) Date of publication of application: **08.02.2017**
(21) Application number: 16183178.9
(22) Date of filing: 08.08.2016
(51) Int. Cl.: A61B 5/08, A61B 5/00

(54) **PHYSIOLOGICAL INFORMATION MEASURING APPARATUS, RESPIRATION INTERVAL DISPLAYING METHOD, AND PROGRAM**

(30) Priority: 07.08.2015 JP 2015157211; 01.08.2016 JP 2016150905
(71) Applicant: Nihon Kohden Corporation, Shinjuku-ku Tokyo (JP)
(72) Inventor: Aoki, Toshiki, Tokyo (JP); Inoue, Masayuki, Tokyo (JP); Hagiwara, Hiroko, Saitama (JP)
(74) Representative: Hoffmann Eitle

(57) **Abstract**

A physiological information measuring apparatus includes: a display controller that, based on information indicating a respiration interval of a patient, is configured to produce a respiration interval graph in which a first axis indicates time information, and a second axis indicates a length of the respiration interval, and that is configured to control a displaying section to display the respiration interval graph, the display controller that is configured to reset a value of the second axis of the respiration interval graph, at each time when the respiration interval starts.

## Description

### BACKGROUND

The presently disclosed subject matter relates to a physiological information measuring apparatus, a respiration interval displaying information measuring method, and a program.

Various apparatuses and methods for monitoring the respiration of the patient who must undergo respiration management in clinical practice have been proposed. For example, the method which is called capnometry is available in which a temporal change of the partial pressure of carbon dioxide contained in the expiration of the patient, i.e., the concentration of carbon dioxide (CO₂ concentration) in the expiration is measured to know the respiration condition of the patient (for example, see JP-T-2003-532992). Also another method is performed in which the expiration and inspiration of the patient are detected by using a flow sensor to know the respiration condition of the patient.

A related-art respiration monitoring apparatus has a function of detecting the respiration interval (or the apneic period) from respiration information which is acquired from a capnometer or a flow sensor. Such a respiration monitoring apparatus outputs an alarm sound when the respiration interval is equal to or larger than a predetermined value, thereby managing the respiration condition of the patient.

As described above, a respiration monitoring apparatus outputs an alarm related to the respiration interval. Even in the case where such a respiration monitoring apparatus is used, however, the doctor or the like cannot know information indicating how the respiration interval of the patient transitions (information indicating, for example, that the respiration interval is being gradually lengthened). Therefore, there is a possibility that deterioration of the respiration condition of the patient progresses.

### SUMMARY

The presently disclosed subject matter may provide a physiological information measuring apparatus, respiration interval displaying method, and program which enable the respiration interval of the patient to be intuitively known.

The physiological information measuring apparatus may comprise: a display controller that, based on information indicating a respiration interval of a patient, is configured to produce a respiration interval graph in which a first axis indicates time information, and a second axis indicates a length of the respiration interval, and that is configured to control a displaying section to display the respiration interval graph, the display controller that is configured to reset a value of the second axis of the respiration interval graph, at each time when the respiration interval starts.

### BRIEF DESCRIPTION OF THE DRAWINGS

Fig. 1 is a block diagram schematically illustrating the configuration of a physiological information measuring apparatus 1.
Fig. 2 is a view illustrating a respiration interval graph which is displayed on a displaying section 13.
Fig. 3 is a block diagram schematically illustrating the configuration of a physiological information measuring apparatus 1 of Embodiment 1.
Fig. 4 is a view illustrating a respiration interval graph which is displayed on a displaying section 13 in Embodiment 1.
Fig. 5 is a view illustrating a respiration interval graph which is displayed on the displaying section 13 in Embodiment 1.
Fig. 6 is a view illustrating the operation of an abnormality detector 15 in Embodiment 1.

### DETAILED DESCRIPTION OF EXEMPLARY EMBODIMENTS

### <Schematic configuration>

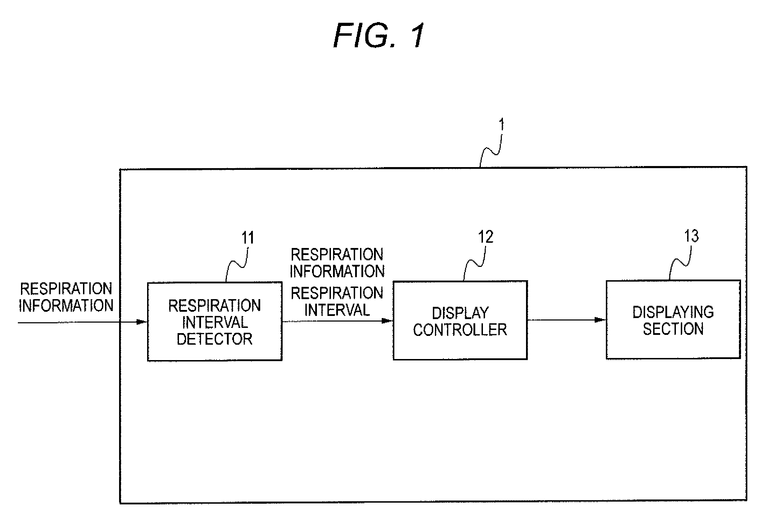

Fig. 1 is a block diagram schematically illustrating the configuration of a physiological information measuring apparatus 1. The physiological information measuring apparatus 1 may include a respiration interval detector 11, a display controller 12, and a displaying section 13.

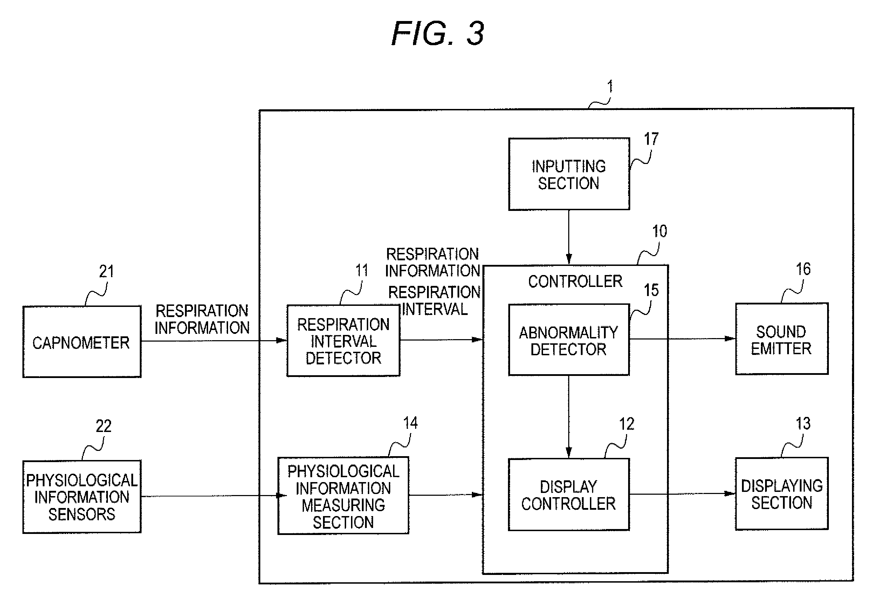

The physiological information measuring apparatus 1 detects the respiration interval of the patient based on respiration information, and displays a respiration interval graph which indicates the length of the respiration interval. The physiological information measuring apparatus 1 may be a single-function apparatus which acquires and displays only respiration information of the patient, or a patient monitor which acquires and displays physiological signals related to various vital signs (such as the arterial oxygen saturation, the body temperature, the blood pressure, the pulse rate, and an electrocardiogram) in addition to respiration information. The configuration of the physiological information measuring apparatus 1 in the case where the apparatus is a patient monitor will be described in detail later with reference to Fig. 3 and other figures.

The physiological information measuring apparatus 1 acquires respiration information. The respiration information is information from which the starts of the expiration and inspiration of the patient can be detected. The respiration information is acquired by a sensor which is connectable to (or communicable with) the physiological information measuring apparatus 1. The respiration information is information which is produced by, for example, a capnometer, a flow sensor, the impedance method, or a thermistor. Alternatively, the respiration information may be obtained by detecting periodic vibration which can be deemed as respiration, from a moving image that is acquired by imaging the chest of the patient.

The respiration interval detector 11 detects a time interval from the timing when the respiration of the patient starts, to that when the next respiration starts, as the respiration interval. In the case where the respiration information is produced by a capnometer, for example, the respiration interval detector 11 may detect an interval from the timing (start of the expiration state) when the partial pressure of carbon dioxide rises from a value (inspiration state) close to 0 mmHg, to the next timing (start of the expiration state) when the partial pressure rises from the value close to 0 mmHg, as the respiration interval. In the case where the respiration information is produced by a flow sensor, the respiration interval detector 11 may detect a time interval from the timing (start of the inspiration state) when the inspiration flow rises, to the timing (start of the inspiration state) when the next inspiration flow rises, as the respiration interval. Also in the case where other respiration information is used, the respiration interval detector 11 may detect the respiration interval by using a related-art technique. The respiration interval detector 11 supplies the respiration information and the respiration interval to the display controller 12.

The respiration information may contain information of the respiration interval. That is, numerical information of the respiration interval may be input to the physiological information measuring apparatus 1. In this case, the physiological information measuring apparatus 1 is configured so as not to include the respiration interval detector 11.

The display controller 12 controls the display of the displaying section 13. The displaying section 13 is configured by a display device disposed on the physiological information measuring apparatus 1, its peripheral circuits, and the like. The displaying section 13 displays numerals and waveforms indicating physiological information of the patient. The displaying section 13 may be configured so as to be detachable from the physiological information measuring apparatus 1.

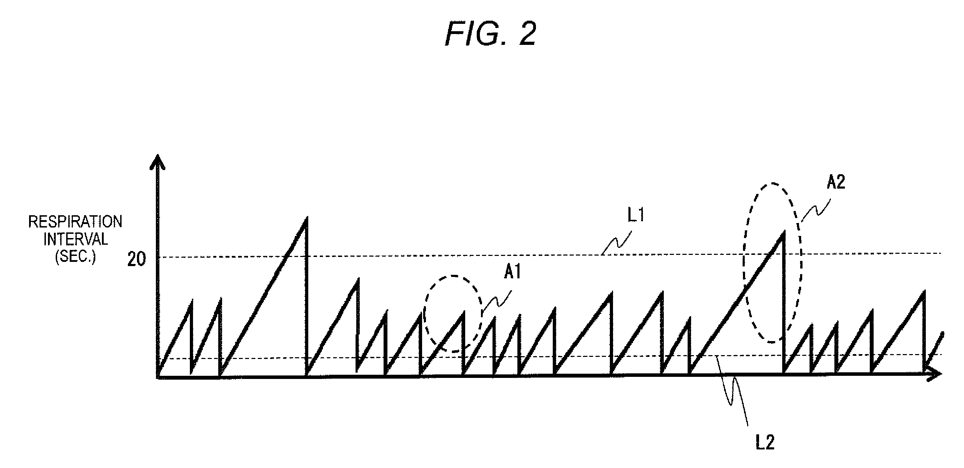

The information of the respiration interval of the patient is supplied to the display controller 12, The display controller 12 produces the respiration interval graph which is to be displayed on the displaying section 13, by using the respiration interval. Fig. 2 is a view illustrating an example of the respiration interval graph which is displayed on the displaying section 13. The display controller 12 produces the respiration interval graph in which the abscissa (first axis) indicates temporal period information (elapsed time period or time information), and the ordinate (second axis) indicates the length of the respiration interval.

The display controller 12 increases, from a certain start timing of respiration, the value of the respiration interval (the value of the ordinate, in other words, the number of seconds), and, at the next start timing of respiration, resets the value (the value of the ordinate) of the respiration interval. As illustrated in Fig. 2, namely, the display controller 12 sets the value of the respiration interval (the value of the ordinate) to 0 at each start of the respiration interval (in other words, at each end of the respiration interval). The display controller 12 may cause also information indicating an abnormal value (critical area) of the respiration interval (in the example of Fig. 2, lines L1 and L2 indicating boundaries between a normal value and an abnormal value), to be displayed in the respiration interval graph. As the information indicating the abnormal value of the respiration interval, both information indicating an upper limit of the respiration interval (the line L1) and information indicating a lower limit of the respiration interval (the line L2) may be displayed, as shown in Fig. 2. Only one of them may be displayed. In the case where the respiration interval exceeds the line L1, it is indicated that the respiration interval is too long. On the other hand, in the case where the respiration interval falls below the line L2, it is indicated that the respiration interval is too short.

The relationship between the abscissa and the ordinate may be reversed (i.e., the ordinate indicates the elapsed time period or the time information).

When the value of the respiration interval (the value of the ordinate) is reset at each respiration, as illustrated in Fig. 2, a state where the respiration interval from certain respiration to next respiration can be visually known is obtained. When referring to the respiration interval graph illustrated in Fig. 2, the doctor or the nurse can know how the respiration interval of the patient transitions. In the example of Fig. 2, the doctor or the like can easily know that, at the timing A1 of Fig. 2, the respiration interval has a normal value, and, at the timing A2 of Fig. 2, the respiration interval has an abnormal value. The doctor or the like can further know the state where the respiration interval is not in the critical area, but the increasing trend continues. Therefore, the doctor or the like can promptly perform a procedure for a respiration abnormality of the patient.

Although, in the above, the description has been made while regarding the time period from a certain start of respiration to the next start of respiration, as the respiration interval, the presently disclosed subject matter is not limited to this. For example, the physiological information measuring apparatus 1 may produce graphs while the expiration time period and the inspiration time period are separated from each other. In this case, the value of the respiration interval (the value of the ordinate) is reset to 0 at the timing of starting the expiration state, and at that of starting the inspiration state. Namely, the total of the expiration time period and the inspiration time period is the respiration interval. In other words, the display controller 12 resets the value of the respiration interval (the value of the ordinate) at each start of the respiration interval, and also at the timing when the expiration and the inspiration are switched in each respiration interval. Also when referring to this respiration interval graph, the doctor or the like can know how the respiration of the patient changes.

### <Embodiment 1>

A detailed embodiment of the physiological information measuring apparatus 1 illustrated in Fig. 1 will be described. In the embodiment, the physiological information measuring apparatus 1 is a patient monitor which can acquire various vital signs (such as the arterial oxygen saturation, the body temperature, the blood pressure, the pulse rate, and an electrocardiogram). In the following description, the processing sections which are identified by the same names and reference numerals as those of Fig. 1 operate in the same manner as the above-described processing sections unless otherwise indicated.

Fig. 3 is a block diagram illustrating the configuration of a physiological information measuring apparatus 1 of the embodiment. The physiological information measuring apparatus 1 may include a controller 10, the respiration interval detector 11, the displaying section 13, a physiological information measuring section 14, a sound emitter 16, and an inputting section 17. The controller 10 controls a display and alarm based on the acquired respiration interval and various physiological information. The controller 10 may include the display controller 12 and an abnormality detector 15. Although not illustrated, the vital signs measuring apparatus 1 may include a CPU (Central Processing Unit), various memories (such as a hard disk drive and a cache memory), and the like.

The physiological information measuring apparatus 1 is connected to physiological information sensors 22 which acquire various physiological signals from the living body of the patient, respectively. For example, the physiological information sensors 22 are electrocardiogram electrodes, an SpO2 probe, a clinical thermometer, and a blood pressure measurement cuff. The physiological information measuring apparatus 1 is connected also to a sensor for acquiring respiration information of the patient. In the embodiment, the physiological information measuring apparatus 1 is connected to a capnometer 21 which acquires the partial pressure of carbon dioxide from the respiration of the patient. As described above, the physiological information measuring apparatus 1 may be connected to a flow sensor or the like which acquires respiration information.

The inputting section 17 is an input interface which is disposed on the physiological information measuring apparatus 1. For example, the inputting section 17 is configured by buttons, knobs, and the like disposed on the housing of the physiological information measuring apparatus 1. Alternatively, the inputting section 17 may have a configuration in which the inputting section is integrated with the displaying section 13 (i.e., a touch panel). The doctor or the like operates the inputting section 17 to input various preset values and switch over screens.

As described above, the respiration interval detector 11 calculates the respiration interval from the respiration information (in the embodiment, a temporal change of the partial pressure of carbon dioxide). The respiration interval detector 11 supplies the detected respiration interval and respiration information to the controller 10.

The physiological information measuring section 14 calculates measurement values and measurement waveforms of vital signs (such as the arterial oxygen saturation, the body temperature, the blood pressure, the pulse rate, and an electrocardiogram) from the physiological signals acquired by the various physiological information sensors 22. The physiological information measuring section 14 supplies the measurement values and measurement waveforms of the vital signs to the controller 10.

The abnormality detector 15 detects an abnormal state of the respiration of the patient based on the respiration information and the respiration interval. In the case where the respiration interval does not meet a predetermined standard (a standard indicating that a respiratory interval is normal), for example, the abnormality detector 15 determines a respiratory abnormality. In the case where the respiration interval is equal to or longer than a predetermined time period (an upper limit threshold indicating that a respiration interval is too long), or is equal to or shorter than a predetermined time period (a lower limit threshold indicating that a respiration interval is too short), for example, the abnormality detector 15 determines the abnormality. It is not necessary to make the determination based on both the upper limit threshold and the lower limit threshold. The determination may be made based on only one of the thresholds. For example, the abnormality detector 15 may determines the respiratory abnormality only by determining whether the respiratory interval is equal to or larger than the predetermined time period (the upper limit threshold indicating that the respiration interval is too long).

The abnormality detector 15 further detects an abnormal state (a state where an alarm is to be output) of the patient based on the measurement values and measurement waveforms of the vital signs. The abnormality detection process performed by the abnormality detector 15 may be equivalent to an algorithm which is used in a usual patient monitor.

In accordance with the kind and level of the detected abnormality, the abnormality detector 15 outputs an alarm sound from the sound emitter 16. The sound emitter 16 is a speaker which outputs an alarm sound and the like under the control of the controller 10. Moreover, the abnormality detector 15 notifies the display controller 12 of the kind and level of the detected abnormality.

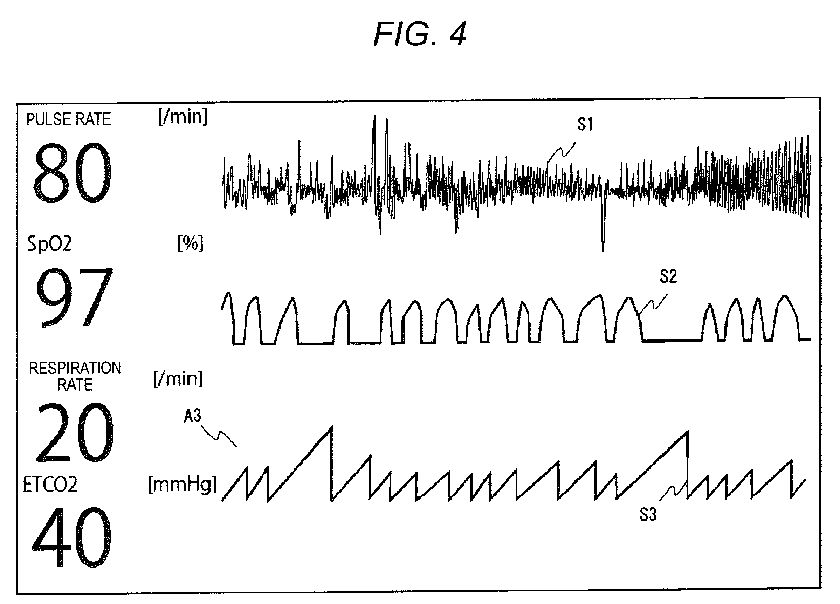

The display controller 12 produces a display screen including the above-described respiration interval graph (corresponding to Fig. 2), and causes the display screen to be displayed on the displaying section 13. Specifically, the display controller 12 produces the display screen which displays in real time the respiration interval graph (corresponding to Fig. 2), and measurement values and measurement waveforms of vital signs, and controls the displaying section 13 so as to display the display screen. Fig. 4 illustrates an example of the display screen.

The display screen illustrated in Fig. 4 displays in real time various measurement values and measurement waveforms of the patient. The abscissa indicates the elapsed time period. In Fig. 4, measurement values of the pulse rate, the SpO2, the respiration rate, and the ETCO2 are displayed on the display screen. In Fig. 4, also a pulse waveform S1, a CO2 waveform S2, and a respiration interval graph S3 are displayed on the display screen. The respiration interval graph S3 is a graph in which the abscissa (first axis) indicates the elapsed time period, and the ordinate (second axis) indicates the length of the respiration interval. In the embodiment, the display controller 12 causes a region A3 indicating an abnormal value of the respiration interval to be displayed together with the respiration interval graph S3.

Preferably, the display controller 12 performs the display control so that an abnormal value of the respiration interval is displayed in any method (the lines L1 and L2 in Fig. 2, or the region A3 in Fig. 4) as described above. Information of an abnormal value of the respiration interval may be displayed in a plurality of levels. For example, information of one of three regions, i.e., a normal region, a region where attention is needed, and a region of an abnormal value may be displayed together with the respiration interval graph S3.

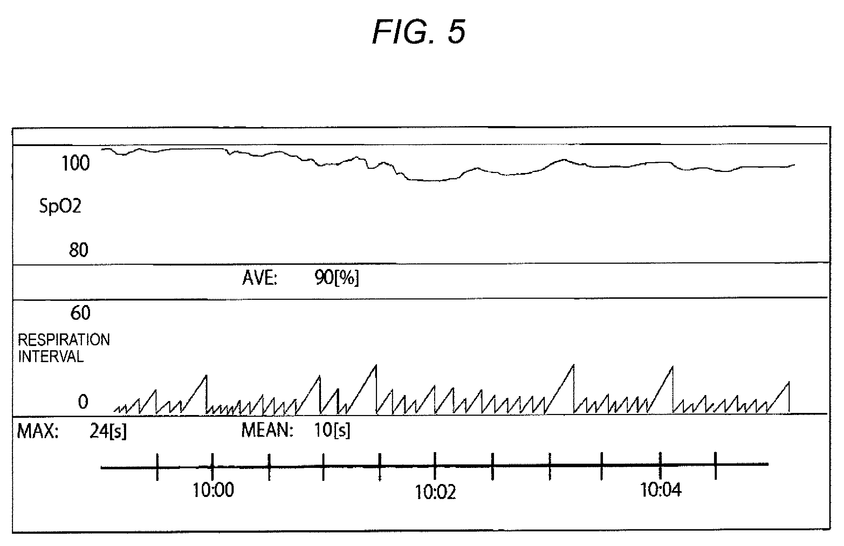

The display controller 12 may cause the history of the respiration interval to be stored in a storage section (such as a hard disk drive) which is not shown, and the respiration interval to be displayed in the form of a long-term waveform (trend graph). An example of the display will be described with reference to Fig. 5.

Fig. 5 illustrates a long-term waveform which displays the transition of the respiration interval of the patient and that of the SpO2 during a period of time from 10:00 to 10:05. The doctor or the like inputs information of a time when the graph and the like are to be displayed, through the inputting section 17. Based on the time information, the display controller 12 gets necessary history information of the respiration interval, and measurement history information of vital signs (in the example, the SpO2) from the storage section.

Then, the display controller 12 produces the display screen (Fig. 5) of the long-term waveform (trend graph) by using the got history information of the respiration interval and measurement history information of the vital signs. In the respiration interval graph (trend graph) illustrated in Fig. 5, the time information is displayed in the abscissa (first axis), and the length of the respiration interval is displayed in the ordinate (second axis).

When referring to the trend graph, the doctor or the like can know how the respiration interval of the patient transitions in the long term.

Then, effects of the physiological information measuring apparatus 1 of the embodiment will be described. The display controller 12 causes the respiration interval graph (Figs. 4 and 5) in which the value is reset at each start of the respiration interval (in other words, at each end of the respiration interval), to be displayed. In the case where the respiration interval is short, the value is frequently reset, and therefore a state where the value of the respiration interval (the value of the ordinate) is small is continued. In the case where the respiration interval is long, by contrast, the value is not frequently reset, and therefore a state where the value of the respiration interval (the value of the ordinate) is large is caused. When referring to the respiration interval graph (Figs. 4 and 5), the doctor or the like can visually know a change of the respiration interval of the patient.

The display controller 12 causes information of an abnormal value of the respiration interval (for example, the region A3 in Fig. 4) to be displayed in the respiration interval graph. Therefore, the doctor or the like can know at a glance whether the respiration interval of the patient is abnormal or not.

The display controller 12 can cause also measurement waveforms and values of other vital signs to be displayed together with the respiration interval graph (Figs. 4 and 5). Therefore, the doctor or the like can know relationships between a change of the respiration interval and changes of the other vital signs.

### <Modifications of abnormality detection>

Hereinafter, modifications of the process in which an abnormality of the respiration interval is detected by the abnormality detector 15 will be described. The abnormality detector 15 may detect an abnormality with considering not only whether the respiration interval exceeds a predetermined value (for example, 20 seconds) or not, but also transitions of the respiration interval. Hereinafter, a modification of the process in which an abnormality of the respiration interval is detected by the abnormality detector 15 will be described.

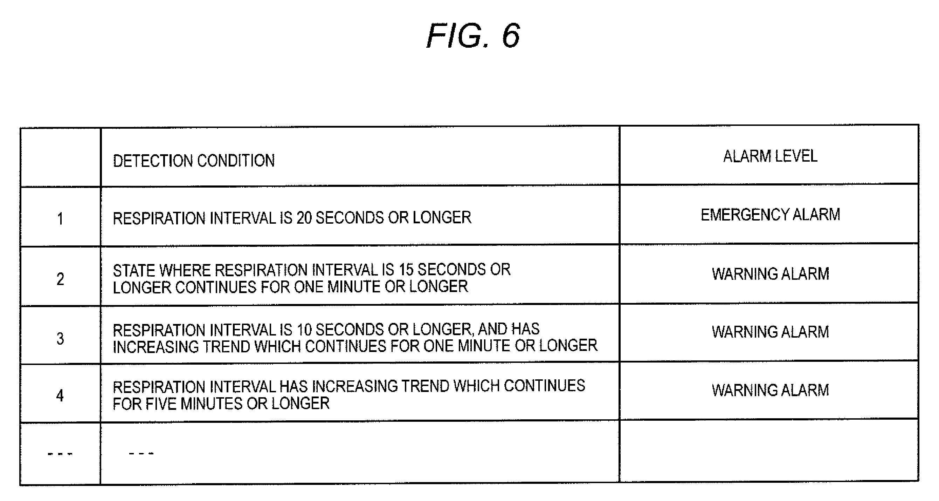

Fig. 6 is a view illustrating examples of detection rules for detecting an abnormality of the respiration interval of the patient. In the example of Fig. 6, four detection rules are set. An alarm level is set for each of the detection rules. In the case where the respiration interval of the patient is 20 seconds or longer (No. 1 of Fig. 6), for example, the alarm level is set as an emergency alarm. Similarly, in the case where a state where the respiration interval of the patient is 15 seconds or longer continues for one minute or longer (No. 2 of Fig. 6), the alarm level is set as a warning alarm. In the case where the respiration interval is 10 seconds or longer, and an increasing trend continues for one minute or longer (No. 3 of Fig. 6), the alarm level is set as a warning alarm. In the case where the respiration interval has an increasing trend which continues for five minutes or longer (No. 4 of Fig. 6), the alarm level is set as a warning alarm. As described above, the abnormality detector 15 detects an abnormality based on not only whether the respiration interval exceeds a predetermined value (for example, 20 seconds) or not (No. 1 of Fig. 6), but also the changing of the respiration interval (Nos. 2 to 4 of Fig. 6).

The abnormality detector 15 detects an abnormality based on the detection rules, and the respiration interval which is detected by the respiration interval detector 11. If a respiration abnormality is detected, the abnormality detector 15 outputs an alarm sound from the sound emitter 16 in accordance with the alarm level. Moreover, the display controller 12 controls the display on the display screen, and the lighting of an indicator lamp in accordance with the alarm level.

Detection rules maybe set as default of the physiological information measuring apparatus 1. The doctor or the like may be allowed to arbitrarily newly register or change detection rules through the inputting section 17.

When the doctor or the like can arbitrarily set detection rules as described above, an abnormality of the respiration interval can be detected with considering the anamnesis and the like of the patient. When an abnormality of the respiration interval is detected with considering the changing of the respiration interval (for example, an increasing trend, a state where the respiration interval is long continues for a predetermined time period, or the like) as illustrated in Nos. 2 to 4 of Fig. 6, deterioration of the respiration interval of the patient can be quickly noticed.

Although the presently disclosed subject matter has been specifically described based on the embodiment, the presently disclosed subject matter is not limited to the above-described embodiment, and it is a matter of course that various changes can be made without departing from the spirit of the presently disclosed subject matter.

At least a part of the above-described processes of the respiration interval detector 11, the display controller 12, the physiological information measuring section 14, and the abnormality detector 15 may be realized as computer programs which are executed in the physiological information measuring apparatus 1.

The programs may be stored by using a non-transitory computer readable medium of any one of various types, and then supplied to the computer. The non-transitory computer readable medium includes tangible storage media of various types. Examples of the non-transitory computer readable medium are a magnetic recording medium (for example, a flexible disk, a magnetic tape, and a hard disk drive), a magneto-optical recording medium (for example, a magneto-optical disk), a CD-ROM (Read Only Memory), a CD-R, a CD-R/W, a semiconductor memory (for example, a mask ROM, a PROM (Programmable ROM), an EPROM (Erasable PROM), a flash ROM, and a RAM (Random Access Memory)). Alternatively, the programs may be supplied to the computer by means of a transitory computer readable medium of any one of various types. Examples of the transitory computer readable medium include an electrical signal, an optical signal, and an electromagnetic wave. The transitory computer readable medium can supply the programs to the computer through a wired communication path such as a metal wire or an optical fiber, or a wireless communication path.

According to an aspect of the presently disclosed subject matter, the display controller causes the respiration interval graph in which the value is reset at each time when the respiration interval ends, to be displayed. In the case where the respiration interval is short, the value is frequently reset, and therefore a state where the respiration interval has a small value is continued. In the case where the respiration interval is long, by contrast, the value is not frequently reset, and therefore a state where the respiration interval has a large value is caused. When referring to the respiration interval graph, the doctor or the like can visually know a change of the respiration interval of the patient.

## Claims

1. A physiological information measuring apparatus comprising:
a display controller that, based on information indicating a respiration interval of a patient, is configured to produce a respiration interval graph in which a first axis indicates time information, and a second axis indicates a length of the respiration interval, and that is configured to control a displaying section to display the respiration interval graph,
the display controller that is configured to reset a value of the second axis of the respiration interval graph, at each time when the respiration interval starts.

2. The physiological information measuring apparatus according to claim 1, wherein
the display controller is configured to control the display section to display the respiration interval graph together with information indicating an abnormal value.

3. The physiological information measuring apparatus according to claim 1 or 2, further comprising:
a storage section that is configured to store a history of the respiration interval, wherein
the display controller is configured to get the history from the storage section, produce the respiration interval graph, and control the displaying section to display the respiration interval graph.

4. The physiological information measuring apparatus according to any one of claims 1 to 3, further comprising:
an abnormality detector that is configured to determine whether the respiration interval detected by a respiration interval detector is abnormal or not.

5. The physiological information measuring apparatus according to claim 4, wherein
the abnormality detector is configured to detect an abnormality based on whether the respiration interval meets a predetermined standard or not.

6. The physiological information measuring apparatus according to claim 4, wherein
the abnormality detector is configured to detect an abnormality based on a changing of the respiration interval.

7. The physiological information measuring apparatus according to any one of claims 1 to 6, wherein
the display controller is configured to control the displaying section to display the respiration interval graph together with a measurement value or a measurement waveform of a vital sign.

8. The physiological information measuring apparatus according to any one of claims 1 to 7, further comprising:
a respiration interval detector that is configured to detect the respiration interval of the patient based on respiration information which is acquired from a living body of the patient.

9. A respiration interval displaying method comprising:
based on information indicating a respiration interval of a patient, producing a respiration interval graph in which a first axis indicates time information, and a second axis indicates a length of the respiration interval, and controlling a displaying section to display the respiration interval graph; and
resetting a value of the second axis of the respiration interval graph, at each time when the respiration interval starts.

10. A program causing a computer to perform a process, the process comprising:
based on information indicating a respiration interval of a patient, producing a respiration interval graph in which a first axis indicates time information, and a second axis indicates a length of the respiration interval, and controlling a displaying section to display the respiration interval graph; and
resetting a value of the second axis of the respiration interval graph, at each time when the respiration interval starts.
